# EUROPEAN PATENT APPLICATION

(11) **EP 4 723 122 A1**
(43) Date of publication of application: **08.04.2026**
(21) Application number: 24815625.9
(22) Date of filing: 31.05.2024
(51) Int. Cl.: G16C 20/30

(54) **INFORMATION PROCESSING DEVICE, INFORMATION PROCESSING METHOD, AND PROGRAM**

(30) Priority: 01.06.2023 JP 2023091157
(71) Applicant: FUJIFILM Corporation, Tokyo 106-8620 (JP)
(72) Inventor: TATESHITA, Masakazu, Ashigarakami-gun, Kanagawa 258-8577 (JP); HIKIDA, Yasushi, Ashigarakami-gun, Kanagawa 258-8577 (JP); MURAKAMI, Ryoichi, Ashigarakami-gun, Kanagawa 258-8577 (JP); SUGIYAMA, Satoshi, Ashigarakami-gun, Kanagawa 258-8577 (JP)
(74) Representative: HGF
(86) International application number: PCT/JP2024/020081
(87) International publication number: WO 2024/248145

(57) **Abstract**

An information processing apparatus includes a processor, in which the processor executes control of outputting a flowchart that indicates a procedure for obtaining a toxicity evaluation result for a plurality of target compounds and that includes a plurality of processing steps set in advance and arranged in time series and a determination processing step in which a determination condition is changeable and a subsequent path branches according to a determination result, and, in a case where, after the toxicity evaluation result is obtained according to the procedure for at least one target compound, the determination condition in the determination processing step is changed and the toxicity evaluation result is obtained under the changed determination condition, in the control of outputting the flowchart, in a case where a change occurs between an unchanged path taken to reach the toxicity evaluation result before the change in the determination condition and a changed path taken to reach the toxicity evaluation result after the change in the determination condition, the processor displays the path in which the change has occurred in a distinguishable manner.

## Description

### Technical Field

The technology of the present disclosure relates to an information processing apparatus, an information processing method, and a program.

### Background Art

In recent years, in the field of handling chemical substances, a toxicity evaluation using a computer-based in silico method has become common. The toxicity evaluation of the chemical substance requires, in addition to simply obtaining the toxicity evaluation, various examinations from the viewpoint of causes of toxicity manifestation or a tendency of a compound that is likely to manifest toxicity.

JP2013-522649A discloses a method of predicting teratogenicity of a test compound, the method comprising: (a) a step of culturing hSLCs in the presence of (i) a first known teratogenic compound and in the absence of (ii) the first known teratogenic compound; (b) a step of detecting a plurality of metabolites having a molecular weight of less than about 3,000 Daltons associated with the hSLCs exposed to the first known teratogenic compound, as compared with the hSLCs not exposed to the first known teratogenic compound, and identifying a difference in a metabolic response of the hSLCs exposed to the first known teratogenic compound as compared with the hSLCs not exposed to the first known teratogenic compound; (c) a step of analyzing the difference in the metabolic response and generating a set of mass characteristics associated with the exposure of the hSLCs to the first known teratogenic compound; (d) a step of repeating the steps (a) to (c) a plurality of times with a plurality of different known teratogenic compounds; (e) a step of classifying the mass characteristics generated from each exposure to the teratogenic compound and obtaining a first reference profile of the mass characteristics; (f) a step of comparing a profile of the mass characteristics generated from the exposure of the hSLCs to the test compound with the first reference profile and predicting the teratogenicity of the test compound; (g) a step of, in a case where the test compound is predicted to be a teratogenic substance, adding the profile of the mass characteristics to the first reference profile to obtain a second reference profile, in which prediction accuracy of the second reference profile is higher than prediction accuracy of the first reference profile; and (h) a step of repeating the steps (f) and (g) a plurality of times with a plurality of different test compounds to obtain a final reference profile.

WO2011/055820A discloses a support device comprising: a measurement data acquisition unit that acquires a plurality of measurement data obtained under different conditions for each of a plurality of types of metabolites and/or metabolic enzymes; a pathway map data acquisition unit that acquires data representing a metabolic pathway map within a living body; an association strength calculation unit that calculates strength of association between the metabolites and/or metabolic enzymes and each metabolic pathway based on the measurement data acquired by the measurement data acquisition unit; a display unit; and a display content determination unit that determines a content to be displayed on the display unit, in which the display content determination unit can cause the display unit to display a content representing two or more metabolic pathways such that the strength of the association is distinguishable, the support device further comprises a pathway selection input receiving unit that receives a selection input of one of the metabolic pathways from a user in a case where the content representing the two or more metabolic pathways is displayed on the display unit, and the display content determination unit causes the display unit to display the metabolic pathway map for the metabolic pathway selected by the selection input received by the pathway selection input receiving unit, and determines the content to be displayed on the display unit such that an increase or decrease in measurement values indicated by the measurement value data for the metabolites and/or metabolic enzymes present on the metabolic pathway map is distinguishable.

JP2004-93234A discloses a toxicity presence/absence determination system including: a storage unit that stores a simultaneous differential equation based on gene pathway information related to metabolism or a sequence of a toxic substance, a reaction rate coefficient of the simultaneous differential equation, a relationship between linear stability and Jacobian stability according to the reaction rate coefficient, and a determination of presence or absence of toxicity of the toxic substance in a value of the reaction rate coefficient; an input unit that inputs gene expression distribution data to the simultaneous differential equation; and a display unit that obtains the reaction rate coefficient by inputting the gene expression distribution data to the simultaneous differential equation and that displays the determination of the presence or absence of the toxicity of the toxic substance.

### SUMMARY OF INVENTION

In the technologies disclosed in JP2013-522649A, WO2011/055820A, and JP2004-93234A, although a method of a toxicity evaluation is considered, an evaluation using a toxicity determination flowchart (hereinafter, also simply referred to as a "toxicity determination flow") used for the toxicity evaluation is not considered. The toxicity determination flow includes a plurality of determination steps, and a path of the subsequent toxicity evaluation branches according to a determination result in each determination step.

In order to improve the accuracy of the toxicity evaluation, a user may change determination conditions of the determination steps in the toxicity determination flow. However, for example, in a case where the toxicity determination flow is complicated, it is difficult to understand an influence of changing the determination conditions of the toxicity determination flow on the evaluation result.

One embodiment according to the technology of the present disclosure provides an information processing apparatus, an information processing method, and a program that, in a case where a determination condition in a toxicity evaluation is changed, make it possible to understand which path a compound that has changed in an evaluation path after the change has followed in a toxicity determination flow used for the toxicity evaluation.

A first aspect according to the technology of the present disclosure is an information processing apparatus comprising: a processor, in which the processor executes control of outputting a flowchart that indicates a procedure for obtaining a toxicity evaluation result for a plurality of target compounds, which are targets of a toxicity evaluation, and that includes a plurality of processing steps set in advance and arranged in time series, the processing steps including a determination processing step in which a determination condition is changeable and a subsequent path branches according to a determination result, and, in a case where, after the toxicity evaluation result is obtained according to the procedure for at least one of the plurality of target compounds, the determination condition in the determination processing step is changed and the toxicity evaluation result is obtained according to the procedure under the changed determination condition, in the control of outputting the flowchart, in a case where a change occurs between an unchanged path taken to reach the toxicity evaluation result before the change in the determination condition and a changed path taken to reach the toxicity evaluation result after the change in the determination condition, the processor displays the path in which the change has occurred in a distinguishable manner.

A second aspect according to the technology of the present disclosure is the information processing apparatus according to the first aspect, in which, in a case where the toxicity evaluation result is obtained for the plurality of target compounds before and after the change in the determination condition, in the flowchart, a display aspect of the path in which the change has occurred is changed according to the number of the target compounds that have passed through the path in which the change has occurred in the toxicity evaluation.

A third aspect according to the technology of the present disclosure is the information processing apparatus according to the second aspect, in which the flowchart shows the plurality of processing steps and connecting lines that connect the processing steps arranged one after the other in time series, and shows the path by using the connecting lines, and the display aspect is a thickness of the connecting line.

A fourth aspect according to the technology of the present disclosure is the information processing apparatus according to the third aspect, in which the thickness of the connecting line increases as the number of the target compounds that have passed through the path in which the change has occurred increases.

A fifth aspect according to the technology of the present disclosure is the information processing apparatus according to the first aspect, in which the flowchart shows the plurality of processing steps and connecting lines that connect the processing steps arranged one after the other in time series, and shows the path by using the connecting lines, and in a case where the processing step or the connecting line shown in the flowchart is selected, an image showing the target compound that has passed through the path shown by the processing step or the connecting line in the toxicity evaluation is displayed.

A sixth aspect according to the technology of the present disclosure is the information processing apparatus according to the fifth aspect, in which the image includes structural data representing a molecular structure of the target compound.

A seventh aspect according to the technology of the present disclosure is the information processing apparatus according to the sixth aspect, in which the image includes structural data representing a common structure that is a partial structure included as a part of the target compound and that is a structure common among the plurality of target compounds that have passed through the selected processing step or connecting line.

An eighth aspect according to the technology of the present disclosure is an information processing method comprising: executing control of outputting a flowchart that indicates a procedure for obtaining a toxicity evaluation result for a plurality of target compounds, which are targets of a toxicity evaluation, and that includes a plurality of processing steps set in advance and arranged in time series, the processing steps including a determination processing step in which a determination condition is changeable and a subsequent path branches according to a determination result, in which, in a case where, after the toxicity evaluation result is obtained according to the procedure for at least one of the plurality of target compounds, the determination condition in the determination processing step is changed and the toxicity evaluation result is obtained according to the procedure under the changed determination condition, in the control of outputting the flowchart, in a case where a change occurs between an unchanged path taken to reach the toxicity evaluation result before the change in the determination condition and a changed path taken to reach the toxicity evaluation result after the change in the determination condition, the path in which the change has occurred is displayed in a distinguishable manner.

A ninth aspect according to the technology of the present disclosure is a program for causing a computer to execute a process comprising: executing control of outputting a flowchart that indicates a procedure for obtaining a toxicity evaluation result for a plurality of target compounds, which are targets of a toxicity evaluation, and that includes a plurality of processing steps set in advance and arranged in time series, the processing steps including a determination processing step in which a determination condition is changeable and a subsequent path branches according to a determination result, in which, in a case where, after the toxicity evaluation result is obtained according to the procedure for at least one of the plurality of target compounds, the determination condition in the determination processing step is changed and the toxicity evaluation result is obtained according to the procedure under the changed determination condition, in the control of outputting the flowchart, in a case where a change occurs between an unchanged path taken to reach the toxicity evaluation result before the change in the determination condition and a changed path taken to reach the toxicity evaluation result after the change in the determination condition, the path in which the change has occurred is displayed in a distinguishable manner.

The technology of the present disclosure provides an information processing apparatus, an information processing method, and a program that, in a case where a determination condition in a toxicity evaluation is changed, make it possible to understand which path a compound that has changed in an evaluation path after the change has followed in a flowchart used for the toxicity evaluation.

### BRIEF DESCRIPTION OF DRAWINGS

FIG. 1 is a conceptual diagram showing a schematic configuration example of an information processing system.
FIG. 2 is a block diagram showing an example of a hardware configuration of an electrical system of a server.
FIG. 3 is a conceptual diagram showing an example of a usage aspect of the information processing system.
FIG. 4 is a functional block diagram showing an example of main functions of a processor of the server.
FIG. 5 is a conceptual diagram showing an example of processing contents of a toxicity evaluation unit.
FIG. 6 is a conceptual diagram showing an example of a usage aspect of the information processing system.
FIG. 7 is a conceptual diagram showing an example of processing contents of a condition setting unit and a toxicity evaluation unit.
FIG. 8 is a conceptual diagram showing an example of processing contents of a difference derivation unit.
FIG. 9 is a conceptual diagram showing an example of processing contents of an image generation unit.
FIG. 10 is a conceptual diagram showing an example of display contents of a display image 64.
FIG. 11 is a conceptual diagram showing an example of an aspect in which a display image is displayed on a display device.
FIG. 12 is a flowchart showing an example of a flow of server control processing.
FIG. 13 is a conceptual diagram showing an example of processing contents of the image generation unit.
FIG. 14 is a conceptual diagram showing an example of an aspect in which a display image is displayed on the display device.
FIG. 15 is a block diagram showing an example of a hardware configuration of an electrical system of a client terminal.
FIG. 16 is a functional block diagram showing an example of main functions of a processor of the client terminal.
FIG. 17 is a functional block diagram showing an example of main functions of the processor of the client terminal.

### DESCRIPTION OF EMBODIMENTS

An example of embodiments of an information processing apparatus, an information processing method, and a program according to the technology of the present disclosure will be described with reference to the accompanying drawings.

### [First Embodiment]

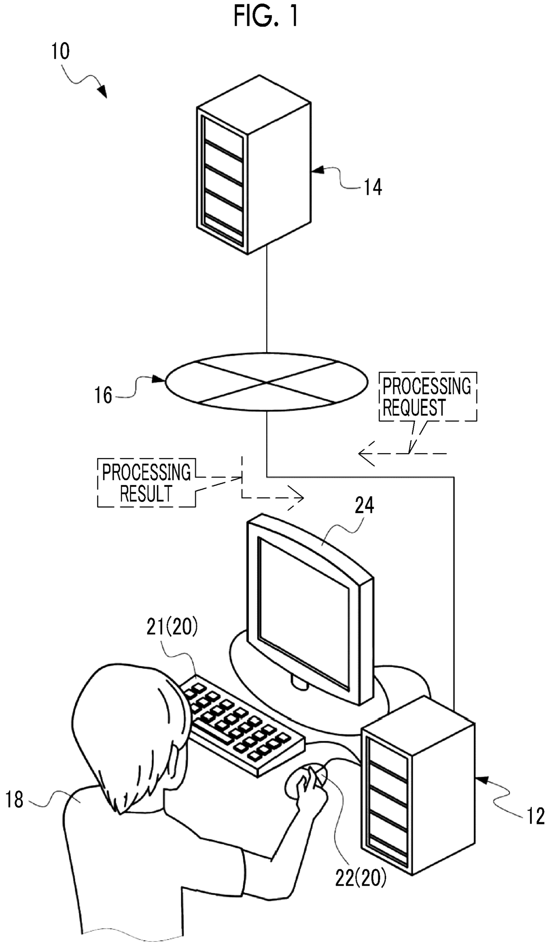

As shown in FIG. 1 as an example, an information processing system 10 comprises a client terminal 12 and a server 14. In the information processing system 10, the client terminal 12 and the server 14 are communicably connected to each other via a network 16.

The information processing system 10 is used, for example, for evaluation of characteristics of a chemical substance (for example, evaluation of the presence or absence of toxicity to a human body). A user 18 (for example, a researcher) can use the information processing system 10 to predict and evaluate experimental results using computer simulations (that is, an evaluation using in silico method). For example, for a newly developed or under-development chemical substance, it is possible to obtain an evaluation result of toxicity without performing a toxicity evaluation test (that is, an in vitro or in vivo toxicity evaluation test) using an actual chemical substance.

The client terminal 12 is a terminal used by the user 18. The server 14 receives a processing request from the client terminal 12 via the network 16 and provides a service corresponding to the request to the client terminal 12 that has made the request via the network 16. For example, in a case where the server 14 receives a request to execute processing related to a characteristic evaluation of a chemical substance as the processing request, the server 14 transmits an evaluation result to the client terminal 12. In the present embodiment, the server 14 is an example of an "information processing apparatus" according to the technology of the present disclosure.

For example, the server 14 is realized by a mainframe, but this is merely an example. For example, the server may be realized by cloud computing, or may be realized by network computing such as fog computing, edge computing, or grid computing. Here, the server 14 is exemplified as an example of a device provided outside the client terminal 12, but this is merely an example, and at least one personal computer or the like may be used instead of the server 14.

The network 16 is configured by, for example, at least one of a wide area network (WAN) or a local area network (LAN). Further, a connection method between the client terminal 12 and the network 16 and a connection method between the server 14 and the network 16 may be a wireless communication method or a wired communication method. The network 16 establishes communication between the client terminal 12 and the server 14, and transmits and receives various types of information between the client terminal 12 and the server 14.

A reception device 20 is connected to the client terminal 12. The reception device 20 receives an instruction from the user 18. The reception device 20 includes a keyboard 21, a mouse 22, and the like. The keyboard 21 and the mouse 22 shown in FIG. 1 are merely an example. As the reception device 20, any one of the keyboard 21 or the mouse 22 may be provided. In addition, as the reception device 20, for example, at least one of a proximity input device that receives a proximity input, a voice input device that receives a voice input, or a gesture input device that receives a gesture input may be applied instead of the keyboard 21 and/or the mouse 22. The proximity input device is, for example, a touch panel or a tablet.

A display device 24 is connected to the client terminal 12. Examples of the display device 24 include an electro-luminescence (EL) display and a liquid crystal display. The display device 24 displays various types of information (for example, image and text) under the control of the client terminal 12.

Here, a personal computer is described as an example of the client terminal 12, but this is merely an example. As the client terminal 12, a mobile terminal, such as a smartphone and a tablet terminal, may be used. Here, although an example in which one client terminal 12 is connected to one server 14 via the network 16 has been described, this is merely an example. It is needless to say that the information processing system 10 may include a plurality of client terminals 12 and a plurality of servers 14.

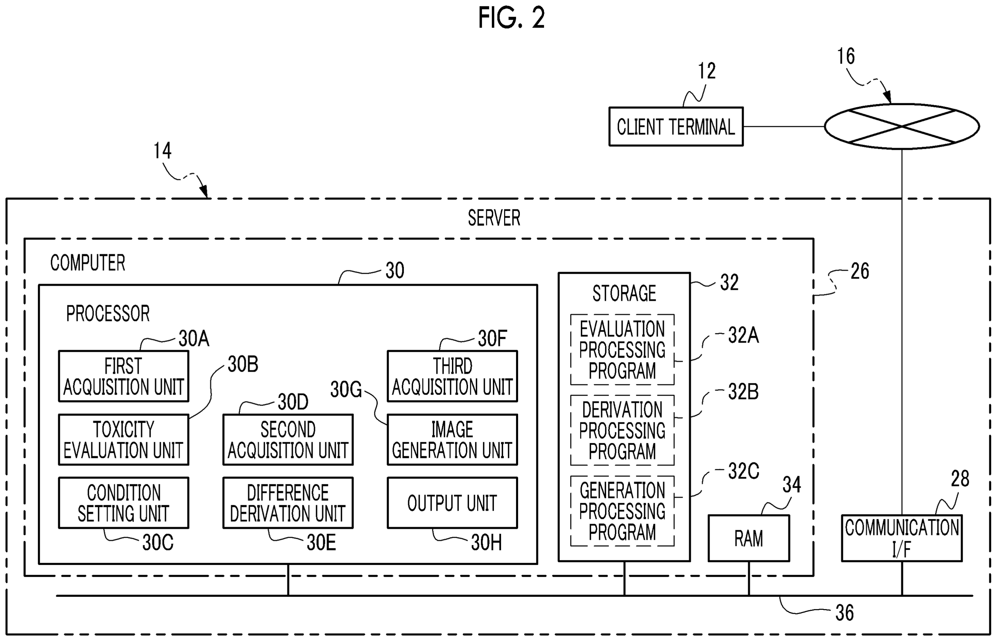

As shown in FIG. 2 as an example, the server 14 comprises a computer 26, a communication interface (I/F) 28, and a bus 36. The computer 26 comprises a processor 30, a storage 32, and a random access memory (RAM) 34. The processor 30, the storage 32, the RAM 34, and the communication I/F 28 are connected to the bus 36. In the present embodiment, the computer 26 is an example of a "computer" according to the technology of the present disclosure, and the processor 30 is an example of a "processor" according to the technology of the present disclosure.

A memory is connected to the processor 30. The memory includes the storage 32 and the RAM 34. The processor 30 includes, for example, a central processing unit (CPU) and a graphics processing unit (GPU). The GPU operates under the control of the CPU, and is responsible for executing processing related to an image.

The storage 32 is a non-volatile storage device that stores various programs, various parameters, and the like. Examples of the storage 32 include a flash memory (for example, an electrically erasable and programmable read only memory (EEPROM) and a solid state drive (SSD)), and/or a hard disk drive (HDD). The flash memory and the HDD are merely an example, and at least one of the flash memory, the HDD, a magnetoresistive memory, or a ferroelectric memory may be used as the storage 32.

The RAM 34 is a memory that transitorily stores information, and is used as a work memory by the processor 30. Examples of the RAM 34 include a dynamic random access memory (DRAM) or a static random access memory (SRAM).

The communication I/F 28 is connected to the network 16. The communication I/F 28 is responsible for transmitting and receiving information to and from an external communication device (for example, the client terminal 12) via the network 16. For example, the communication I/F 28 transmits information in response to a request from the processor 30 to the external communication device via the network 16. In addition, the communication I/F 28 receives the information transmitted from the external communication device, and outputs the received information to the processor 30 via the bus 36.

An evaluation processing program 32A is stored in the storage 32. The evaluation processing program 32A is a program that provides an evaluation simulation of toxicity of a chemical substance. The processor 30 reads out the evaluation processing program 32A from the storage 32 and executes the read-out evaluation processing program 32A on the RAM 34 to perform toxicity evaluation processing. The toxicity evaluation processing is realized by the processor 30 operating as a first acquisition unit 30A, a toxicity evaluation unit 30B, and a condition setting unit 30C.

In addition, a derivation processing program 32B is stored in the storage 32. The derivation processing program 32B is a program for deriving a change in a path in the toxicity evaluation before and after updating a toxicity determination flow. The processor 30 reads out the derivation processing program 32B from the storage 32 and executes the read-out derivation processing program 32B on the RAM 34 to perform difference derivation processing. The difference derivation processing is realized by the processor 30 operating as a second acquisition unit 30D and a difference derivation unit 30E.

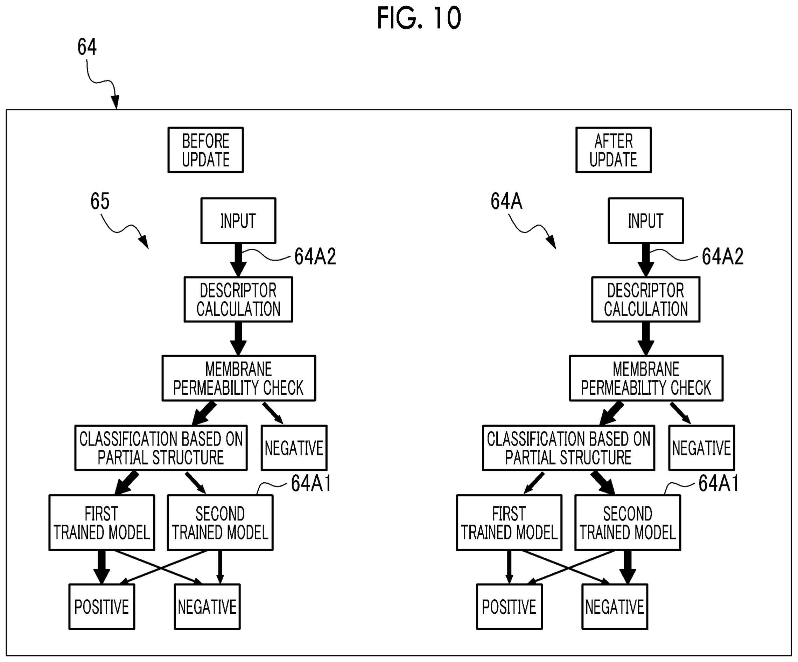

In addition, a generation processing program 32C is stored in the storage 32. The generation processing program 32C is a program that generates a display image 64 (see FIG. 10 and the like) to be output to the client terminal 12. The processor 30 reads out the generation processing program 32C from the storage 32 and executes the read-out generation processing program 32C on the RAM 34 to perform image generation processing. The image generation processing is realized by the processor 30 operating as a third acquisition unit 30F, an image generation unit 30G, and an output unit 30H. In the present embodiment, the generation processing program 32C is an example of a "program" according to the technology of the present disclosure.

Here, a case where the user 18 uses the information processing system 10 to make an evaluation of toxicity to a human body (hereinafter, also simply referred to as a "toxicity evaluation") for a newly developed chemical substance A will be considered. Here, the toxicity refers to toxicity that the user 18 is interested in as an evaluation item, and examples thereof include mutagenicity, sensitization, and irritation. The mutagenicity refers to a property of causing an irreversible change in genetic information (a base sequence of deoxyribonucleic acid (DNA) or a structure or number of chromosomes) of an organism. The sensitization refers to a property of causing an allergic reaction upon exposure to a chemical substance. The irritation refers to a property in which a chemical substance or the like gives a stimulus (for example, pain or a burning sensation) to the tactile sense or the like.

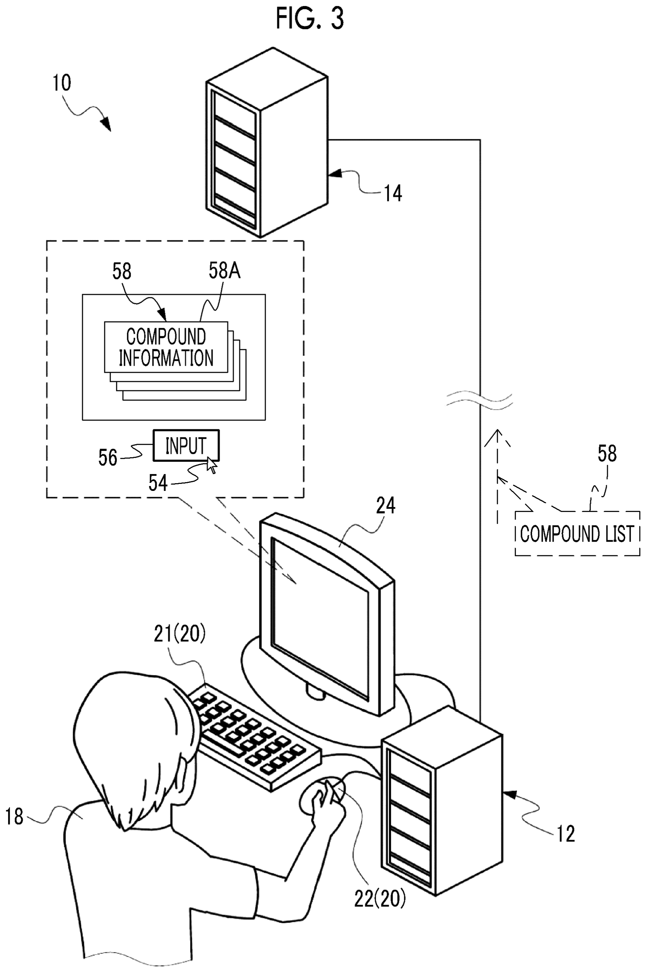

In a case of evaluating the toxicity, as shown in FIG. 3 as an example, the user 18 inputs a compound list 58 to the client terminal 12 via the reception device 20. The compound list 58 is a list of chemical substances that are targets of the toxicity evaluation. As an example, the number of compounds to be subjected to the toxicity evaluation in one toxicity evaluation ranges from several thousand to several tens of thousands. Examples of the chemical substance include a low-molecular-weight compound (for example, a compound having a molecular weight of less than 1000), a sugar, a peptide, a protein, and a nucleic acid. The compound list 58 includes a plurality of pieces of compound information 58A. The compound information 58A is information for specifying a chemical structure of the compound. More specifically, the compound information 58A is text information describing a chemical structure such as a structural formula, a compositional formula, and an amino acid sequence, or numerical information obtained by converting such text information into a numerical value. In addition, the compound list 58 may be a list of information indicating a chemical abstract service (CAS) number or a name of a chemical substance. In this case, the client terminal 12 or the server 14 refers to table data or the like in which a correspondence relationship between a CAS number or a name of a chemical substance and a chemical structure is recorded, and reads out the compound information 58A corresponding to the input chemical substance.

In addition, the compound list 58 may be a list of image data showing a chemical structure of a compound as a compound graph (that is, a data structure in which atoms are nodes and bonds are edges). In this case, the client terminal 12 or the server 14 refers to table data or the like in which a correspondence relationship between the compound graph and the compound information 58A is recorded, and reads out the compound information 58A corresponding to the selected compound graph.

In the example shown in FIG. 3, a screen for selecting a compound list 58 is displayed on a screen of the display device 24, and the user 18 performs a selection operation on the selection screen to select, for example, the compound list 58. On the selection screen, for example, the user 18 clicks an input soft key 56 by operating a pointer 54 displayed on the screen of the display device 24 via the mouse 22. As a result, the compound list 58 is transmitted from the client terminal 12 to the server 14, and a processing request is made regarding the toxicity evaluation for the compound.

Here, although an example of a form in which the plurality of pieces of compound information 58A are listed and input at once has been described, it is needless to say that each of the plurality of pieces of compound information 58A may be individually input or may be input by being divided into a plurality of lists. In addition, one piece of compound information 58A may be input, and the toxicity of one compound and metabolites may be evaluated.

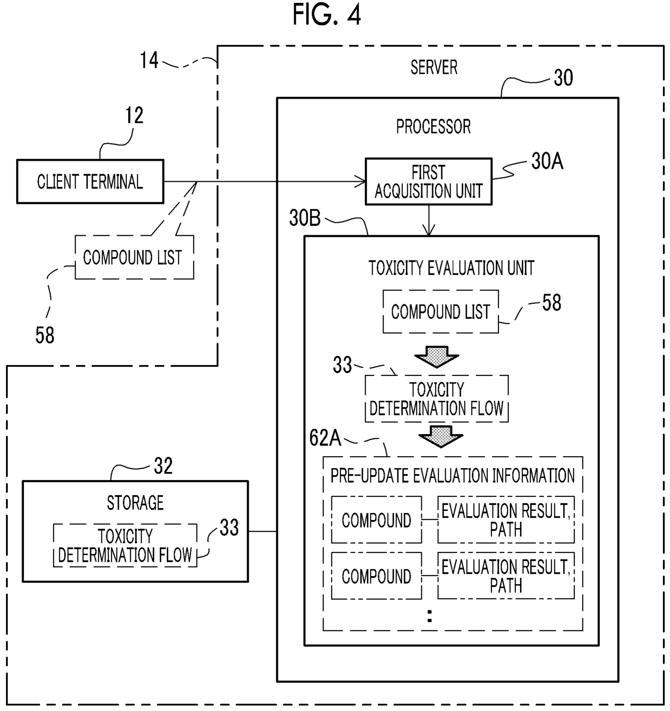

As shown in FIG. 4 as an example, the compound list 58 is output from the client terminal 12 to the server 14. In the processor 30 of the server 14, the toxicity evaluation processing is executed. The toxicity evaluation processing is processing of evaluating the toxicity of a compound. In the toxicity evaluation processing, the first acquisition unit 30A outputs the acquired compound list 58 to the toxicity evaluation unit 30B. The toxicity evaluation unit 30B evaluates the toxicity of a plurality of compounds (that is, a compound group) indicated by the compound information 58A of the compound list 58.

Specifically, the toxicity evaluation unit 30B executes the toxicity evaluation processing according to a toxicity determination flow 33. The toxicity determination flow 33 is described in the evaluation processing program 32A, and is schematically shown in a form of being stored in the storage 32 in FIG. 4. The toxicity determination flow 33 is a determination flow used for evaluating the toxicity.

The toxicity determination flow 33 includes a plurality of processing steps. The plurality of processing steps are set in advance and are arranged in time series along a procedure of the toxicity evaluation. In addition, the processing steps include a determination processing step, and a subsequent path branches according to a determination result in the determination processing step. Determination items in each determination processing step are predetermined. Examples of the determination item include whether or not a compound permeates a cell membrane, and whether or not a compound corresponds to a partial structure rule. The partial structure rule is a regulation related to a chemical structure, and is a rule for specifying a predetermined partial structure (for example, a partial structure of interest to the user 18). The partial structure rule is, for example, the presence or absence of a benzene ring in the chemical structure and the number of benzene rings. In addition, a determination condition in the determination processing step is changeable.

The toxicity evaluation unit 30B inputs the compound list 58 to the toxicity determination flow 33. The toxicity evaluation unit 30B executes a determination regarding toxicity evaluation for each determination step on the compound information 58A in the toxicity determination flow 33. The toxicity evaluation unit 30B outputs pre-update evaluation information 62A as toxicity evaluation results according to the toxicity determination flow 33 to be executed. Steps ST1 to ST5 described below are examples of a "processing step" according to the technology of the present disclosure, and steps ST2 to ST5 are examples of a "determination processing step" according to the technology of the present disclosure.

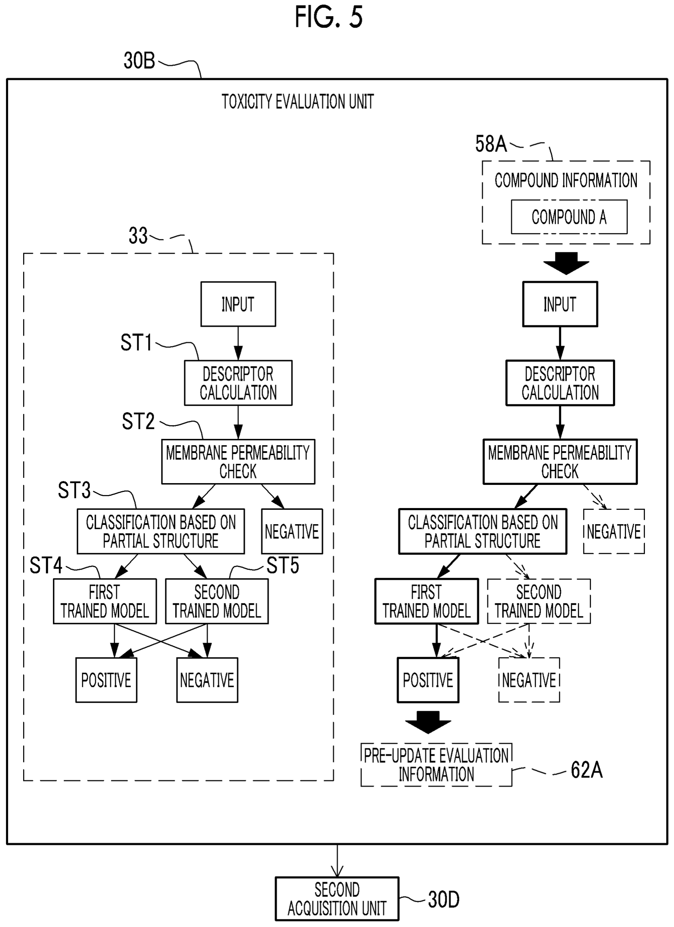

As shown in FIG. 5 as an example, the compound information 58A is input to the toxicity determination flow 33. In the toxicity determination flow 33, in step ST1, descriptor calculation is executed on the compound information 58A. Examples of the descriptor include a molar mass or a LogP (a value obtained by taking the common logarithm of an octanol/water partition coefficient) of the compound. After the process of step ST2 is executed, the toxicity evaluation proceeds to step ST3.

In step ST2, it is determined whether or not the compound can permeate the cell membrane based on a result of the descriptor calculation of the compound executed in step ST1. The numerical value indicated by the descriptor is compared with a threshold value, and a determination is made as to whether or not the compound can permeate the cell membrane based on a comparison result. In a case where the determination in step ST2 is affirmative, the toxicity evaluation proceeds to step ST3. In a case where the determination in step ST2 is negative, a negative (that is, non-toxic) evaluation result is output as the result of the toxicity evaluation. This is because the compound does not permeate the cell membrane and is therefore considered to have little effect on the body.

In step ST3, the compounds are classified based on a partial structure of the compound in step ST3. Specifically, the partial structure rule is applied to the compound information 58A. As a result, it is determined whether or not the compound has the partial structure. Then, the compounds are classified based on a determination result as to whether or not a molecular structure of each compound includes the partial structure. That is, the compounds are sorted into a predetermined category according to a combination of the partial structures of the compound. There may be a plurality of partial structure rules, and, for each of the plurality of partial structure rules, it is determined whether or not the compound that is the target of the toxicity evaluation has the partial structure. After the process of step ST3 is executed, the toxicity evaluation proceeds to step ST4.

The toxicity evaluation proceeds to step ST4 according to the classification result in step ST3. In step ST4, determination is made using a first trained model. The first trained model realizes a function of determining toxicity, for example, by training a neural network through machine learning using training data. An example of training data is a data set obtained from results of past experiments, in which information for specifying a classified test substance is used as example data, and information for specifying the determination result of toxicity is used as correct answer data.

In addition, the toxicity evaluation proceeds to step ST5 according to the classification result in step ST3. In step ST5, determination is made using a second trained model. The second trained model is a trained model different from the first trained model. The second trained model realizes a function of determining toxicity, for example, by training a neural network through machine learning using training data. An example of training data is a data set obtained from results of past experiments, in which information for specifying a classified test substance is used as example data, and information for specifying the determination result of toxicity is used as correct answer data.

A final toxicity evaluation result is output through the determinations in step ST4 and step ST5. As the toxicity evaluation result, a positive (that is, toxic) or negative (that is, non-toxic) evaluation result is output.

Here, although an example has been described in which a positive or negative value (for example, a value of 1 for positive and 0 for negative) is output as the toxicity evaluation result, this is merely an example. As the toxicity evaluation result, a value (for example, a score between 0 and 1) indicating a probability of being positive or negative may be output.

In the example shown in FIG. 5, paths and processing steps taken in the toxicity determination flow 33 are shown as the toxicity evaluation for a compound A, and an example in which the compound A is finally evaluated as positive is shown. In addition, the pre-update evaluation information 62A includes information indicating the result of the toxicity evaluation as well as information indicating a path (hereinafter, also simply referred to as an "evaluation path") leading to the result of the toxicity evaluation in the toxicity determination flow 33, information indicating an explanation of the determination item, and information indicating details of a determination result for each processing step.

As described above, the toxicity determination flow 33 outputs the pre-update evaluation information 62A according to the input compound information 58A. In the pre-update evaluation information 62A, the evaluation result and the path are linked to each compound (see FIG. 4). In this way, the toxicity evaluation processing is executed. The toxicity evaluation unit 30B outputs the pre-update evaluation information 62A to the second acquisition unit 30D.

Incidentally, in a case where the toxicity evaluation of the compound is performed using the toxicity determination flow 33, the user may change the determination condition in the determination processing step. The purpose of changing the determination condition is, for example, to improve the accuracy of the toxicity evaluation. For example, the user considers the evaluation result obtained by using a certain toxicity determination flow 33, and performs adjustment (for example, adding a partial structure rule or changing a threshold value related to membrane permeability) of the determination condition based on organic chemical theory. As a result, the accuracy of the toxicity evaluation by the updated toxicity determination flow 33 may be improved.

However, in a case where the toxicity determination flow 33 is updated, it is difficult to understand the effect of the update on the toxicity evaluation by the updated toxicity determination flow 33. For example, in a case where the toxicity determination flow 33 is complicated (for example, in a case where there are a large number of determination processing steps), it is difficult to understand how the change in the determination processing step affects the change in the evaluation result.

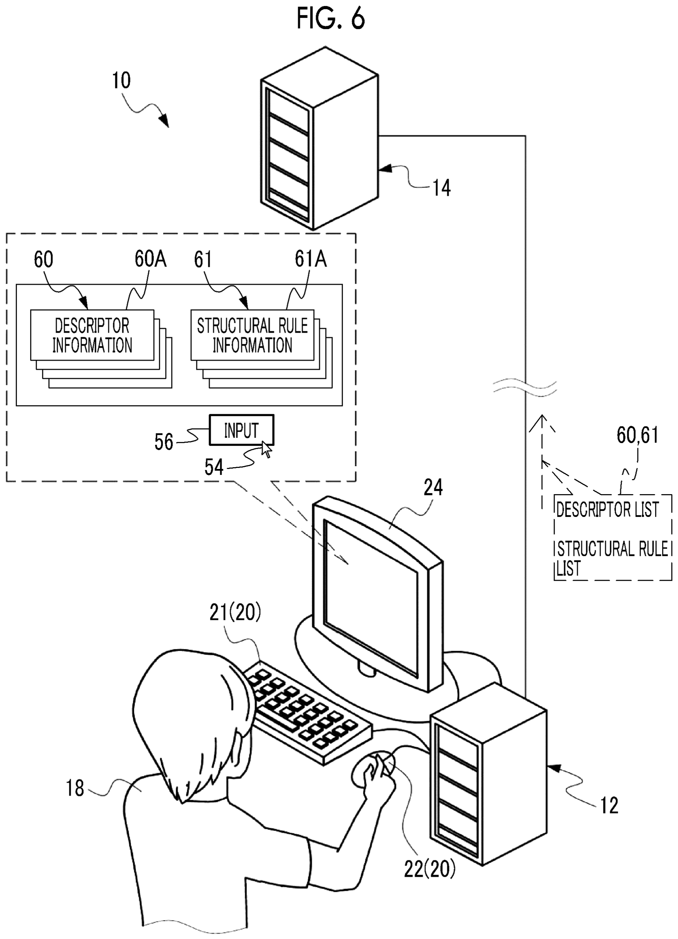

Therefore, in the present embodiment, the difference derivation processing is executed by the processor 30. The difference derivation processing is processing of deriving a change that has occurred in the toxicity evaluation before and after the update of the toxicity determination flow 33. As shown in FIG. 6 as an example, the user 18 inputs a descriptor list 60 and a structural rule list 61 to the client terminal 12 via the reception device 20. The descriptor list 60 is a list of descriptors used for determination in the determination processing step of the updated toxicity determination flow 33. The descriptor list 60 includes a plurality of pieces of descriptor information 60A. The descriptor information 60A includes information for specifying a descriptor after change, which is used in a processing step (for example, step ST1 shown in FIG. 5) of performing the descriptor calculation. In addition, the descriptor information 60A also includes information for specifying a threshold value after change, which is used in a determination processing step (for example, step ST2 shown in FIG. 5) of comparing the descriptor calculation result with a threshold value.

The structural rule list 61 is a list of partial structure rules. The structural rule list 61 includes a plurality of pieces of structural rule information 61A. The structural rule information 61A is information for specifying a partial structure. More specifically, the structural rule information 61A is text information describing a chemical structure such as a structural formula, a compositional formula, and an amino acid sequence of the partial structure, or numerical information obtained by converting such text information into a numerical value. In addition, the structural rule list 61 may be a list of information indicating a name of a chemical structure. In this case, the client terminal 12 or the server 14 refers to table data or the like in which a correspondence relationship between a name of a chemical structure and a chemical structure is recorded, and reads out the structural rule information 61A corresponding to the input partial structure.

In addition, the structural rule list 61 may be a list of image data showing a chemical structure of a partial structure as a compound graph (that is, a data structure in which atoms are nodes and bonds are edges). In this case, the client terminal 12 or the server 14 refers to table data or the like in which a correspondence relationship between the compound graph and the structural rule information 61A is recorded, and reads out the structural rule information 61A corresponding to the selected compound graph.

In the example shown in FIG. 6, a screen for selecting the descriptor list 60 and the structural rule list 61 is displayed on a screen of the display device 24, and the user 18 performs a selection operation on the selection screen to select, for example, the descriptor list 60 and the structural rule list 61. On the selection screen, for example, the user 18 clicks an input soft key 56 by operating the pointer 54 displayed on the screen of the display device 24 via the mouse 22. As a result, the descriptor list 60 and the structural rule list 61 are transmitted from the client terminal 12 to the server 14, and a processing request is made regarding the update of the toxicity determination flow 33.

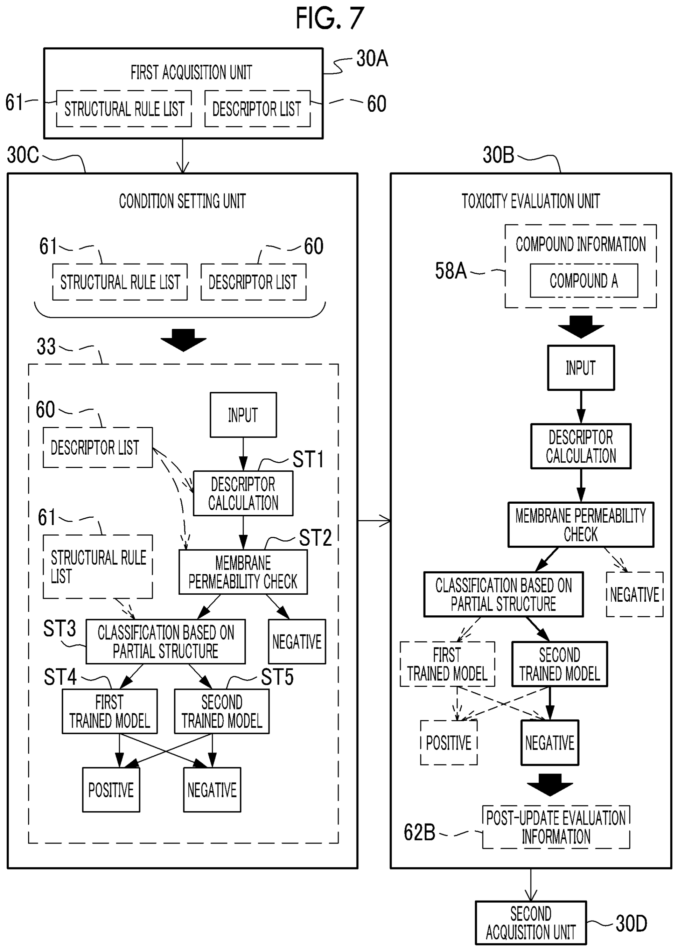

As shown in FIG. 7 as an example, the first acquisition unit 30A outputs the acquired descriptor list 60 and structural rule list 61 to the condition setting unit 30C. The condition setting unit 30C changes the determination condition of the determination processing step of the toxicity determination flow 33 based on the descriptor list 60 and the structural rule list 61. Specifically, the condition setting unit 30C changes a descriptor used in the processing step of executing the descriptor calculation, according to the descriptor information 60A indicated by the descriptor list 60. In addition, the condition setting unit 30C changes a threshold value used in the determination processing step of comparing the descriptor calculation result with a threshold value, according to the descriptor information 60A. In the example shown in FIG. 7, an example is shown in which the descriptor in the step ST1 is changed based on the descriptor list 60, and the threshold value in the membrane permeability check of the step ST2 is further changed.

The condition setting unit 30C changes a partial structural rule used in the determination processing step in which the partial structure rule is applied, according to the structural rule information 61A indicated by the structural rule list 61. In the example shown in FIG. 7, the partial structure rule applied in the step ST3 is changed based on the structural rule list 61. For example, in a case where a benzene ring is exemplified as the partial structure, the structural formula determined as a benzene ring by the structural rule list 61 is changed. Then, the condition setting unit 30C outputs the updated toxicity determination flow 33 to the toxicity evaluation unit 30B.

The toxicity evaluation unit 30B uses the updated toxicity determination flow 33 to evaluate the toxicity of a plurality of compounds (that is, a compound group) indicated by the compound information 58A of the compound list 58. Here, the compound group to be evaluated is the same as the compound group for which the toxicity evaluation has been performed according to the pre-update toxicity determination flow 33. The toxicity evaluation unit 30B outputs the result of the toxicity evaluation performed by using the updated toxicity determination flow 33 to the second acquisition unit 30D as post-update evaluation information 62B. As with the pre-update evaluation information 62A, the post-update evaluation information 62B includes, in addition to the information indicating the result of the toxicity evaluation, information indicating the evaluation path, information indicating the description of the determination item, and information indicating the details of the determination result for each processing step.

In the example shown in FIG. 7, paths and determination steps taken in the updated toxicity determination flow 33 are shown as the toxicity evaluation for a compound A, and an example in which the compound A is finally evaluated as negative is shown. Here, the evaluation path in the toxicity evaluation of the compound A shown in FIG. 7 is different from the evaluation path in the toxicity evaluation of the compound A shown in FIG. 5. Specifically, the change in the determination condition in step ST3 results in a different determination result for the compound A in step ST3, and a change occurs in the subsequent evaluation path. Further, the toxicity evaluation result of the compound A is positive before the change in the determination condition, but is changed to negative after the update of the determination condition, so that the conclusion of the toxicity evaluation result is also different. As described above, in the toxicity determination flow 33, the path in the toxicity evaluation of the compound may be changed by changing the determination condition of the determination processing step. Here, a case is shown in which a change occurs in the evaluation path in the toxicity determination flow 33 for the compound A, but it is needless to say that a change in the evaluation path may also occur for other compounds.

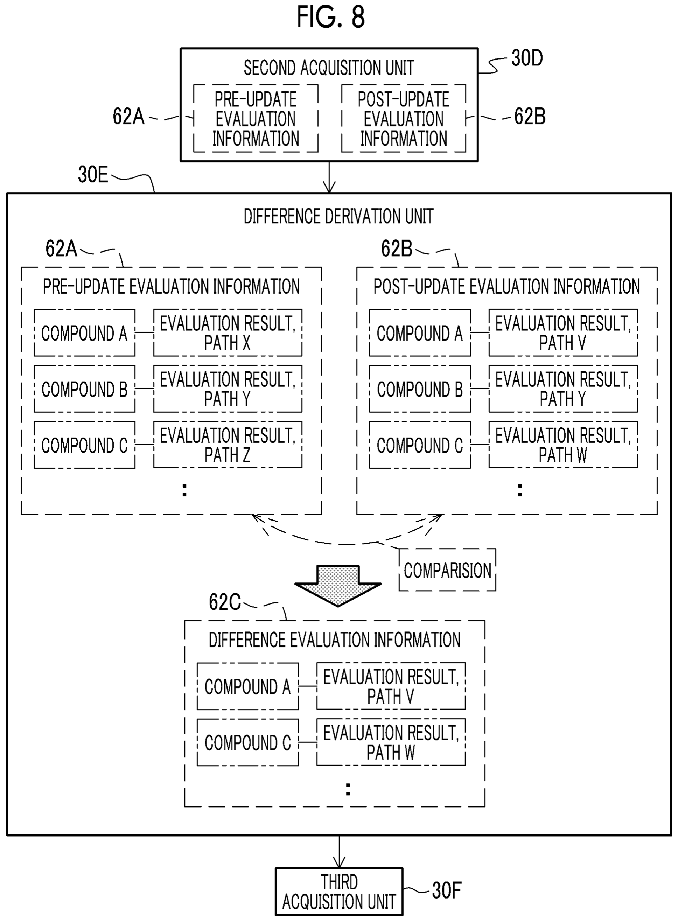

In this case, as an example, as shown in FIG. 8, the second acquisition unit 30D outputs the pre-update evaluation information 62A and the post-update evaluation information 62B to the difference derivation unit 30E. The difference derivation unit 30E executes difference derivation processing. Specifically, the difference derivation unit 30E compares, for each compound, an evaluation path indicated by the pre-update evaluation information 62A with an evaluation path indicated by the post-update evaluation information 62B. Then, the difference derivation unit 30E extracts compounds in the post-update evaluation information 62B that have undergone a change in the evaluation path from the pre-update evaluation information 62A, and generates difference evaluation information 62C. The difference evaluation information 62C is information for specifying a compound whose evaluation path has changed and the changed evaluation path in a case where the determination condition is changed.

In the example shown in FIG. 8, as a result of comparing the pre-update evaluation information 62A with the post-update evaluation information 62B, compounds A and C whose evaluation paths have changed are extracted, and information indicating these compounds and their evaluation paths is generated as the difference evaluation information 62C. Then, the difference derivation unit 30E outputs the difference evaluation information 62C to the third acquisition unit 30F.

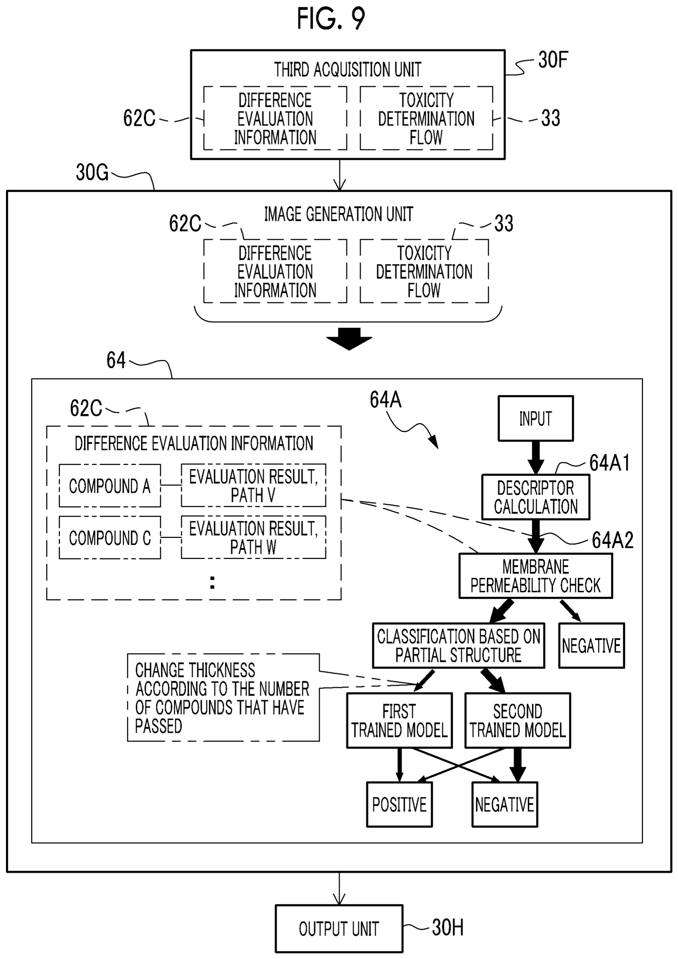

As an example, as shown in FIG. 9, the third acquisition unit 30F outputs the difference evaluation information 62C and the toxicity determination flow 33 to the image generation unit 30G. The image generation unit 30G generates a display image 64 based on the difference evaluation information 62C and the toxicity determination flow 33. The display image 64 includes a flow image 64A. The flow image 64A is an example of a "flowchart" according to the technology of the present disclosure.

The flow image 64A is an image showing a procedure for obtaining a result of the toxicity evaluation of the compound group according to the toxicity determination flow 33. The flow image 64A includes a plurality of step symbols 64A1 and connecting lines 64A2. The connecting lines 64A2 indicate a path of the toxicity evaluation in the toxicity determination flow 33. The step symbols 64A1 are symbols indicating the content of each processing step in the toxicity determination flow 33. Here, as an example, text explaining the content of the processing step is shown within a rectangular frame line.

The connecting lines 64A2 are lines that connect the step symbols 64A1 that are arranged one after the other in time series, and are shown here as arrows as an example. In the flow image 64A, the evaluation path after the change is displayed in a distinguishable manner for a compound whose evaluation path indicated by the difference evaluation information 62C has changed. The connecting line 64A2 is an example of a "connecting line" according to the technology of the present disclosure.

In addition, a display aspect of the evaluation path after the change is changed according to the number of compounds that have passed through the evaluation path after the change. In the example shown in FIG. 9, in the flow image 64A, a thickness of the connecting line 64A2 indicating the evaluation path after the change of a compound whose evaluation path has changed is changed according to the number of compounds that have passed through the connecting line 64A2. As a result, the evaluation path after the change of the compound whose evaluation path has changed is shown. In the example shown in FIG. 9, the thickness of the connecting line 64A2 increases as the number of compounds that have passed through the connecting line 64A2 increases.

That is, as shown in FIG. 9, the difference evaluation information 62C includes the evaluation result and the evaluation path for each compound whose evaluation path has changed, such as the compound A and the compound C. In a case of generating the flow image 64A, as an example, the image generation unit 30G counts the number of compounds that pass through the paths between the processing steps of the toxicity determination flow 33 based on the evaluation path for each compound extracted from the difference evaluation information 62C, and, as the number of compounds is larger, the image generation unit 30G makes the thickness of the connecting line 64A2 corresponding to the path larger.

In the example shown in FIG. 9, first, for compounds whose evaluation paths have changed, the connecting line 64A2 corresponding to the path leading to the "classification based on partial structure" is thicker than the path leading from the processing step of the "membrane permeability check" to a negative result. From this, it can be seen that, among the compounds whose evaluation paths have changed, the number of compounds that pass through the path leading from the processing step of the "membrane permeability check" to the "classification based on partial structure" is large. In addition, the thicknesses of two connecting lines 64A2 branching from the processing step of the "classification based on partial structure" indicate that the number of compounds that pass through the path leading to the "second trained model" is larger than the number of compounds that pass through the path leading to the "first trained model". Then, for compounds whose evaluation paths have changed, the thicknesses of two connecting lines 64A2 leading from the "second trained model" to "positive" and "negative" indicate that there are many compounds that have been determined as "negative" in the "second trained model".

As described above, the difference evaluation information 62C lists only compounds whose evaluation paths have changed before and after the change in the determination condition. As in the example flow image 64A shown in FIG. 9, by changing the thickness of the connecting line 64A2, it is possible to understand what evaluation path most of the compounds followed for which the evaluation path listed in the difference evaluation information 62C has changed.

Here, although an example of a form in which the thickness of the connecting line 64A2 is changed according to the number of compounds that have passed through the path in which the change has occurred has been described, this is merely an example. For example, instead of or in addition to the thickness of the connecting line 64A2, the color of the connecting line 64A2 (for example, the more compounds there are, the redder the color; the fewer compounds there are, the bluer the color) and/or the type of line (for example, the more compounds there are, the more solid the line; the fewer compounds there are, the more dotted the line) may be changed according to the number of compounds.

In addition, in the flow image 64A, each step symbol 64A1 is associated with information indicating the compound that has passed through the processing step based on the difference evaluation information 62C. In addition, each connecting line 64A2 is also associated with information indicating the compound that has passed through the connecting line 64A2 based on the difference evaluation information 62C. Specifically, in the flow image 64A, the compound indicated by the difference evaluation information 62C is associated with the step symbol 64A1 and the connecting line 64A2 corresponding to the evaluation path indicated by the difference evaluation information 62C.

In addition, as shown in FIG. 10 as an example, in the display image 64, a pre-update flow image 65 is displayed together with the flow image 64A. The pre-update flow image 65 is an image showing the evaluation path taken by the compound indicated by the difference evaluation information 62C in the toxicity determination flow 33 (see FIG. 5) before being updated. The image generation unit 30G generates the pre-update flow image 65 based on the pre-update toxicity determination flow 33 and the difference evaluation information 62C, similarly to the flow image 64A. Then, the image generation unit 30G arranges the pre-update flow image 65 and the flow image 64A side by side in the display image 64.

In the pre-update flow image 65 as well, the display aspect of the evaluation path is changed according to the number of compounds that have passed through the evaluation path. In the example shown in FIG. 10, for a compound whose evaluation path has changed, a thickness of the connecting line 64A2 indicating the evaluation path before the change is changed according to the number of compounds that have passed through the connecting line 64A2. As a result, the evaluation path before the change of the compound whose evaluation path has changed is shown. In the example shown in FIG. 10, the thickness of the connecting line 64A2 increases as the number of compounds that have passed through the connecting line 64A2 increases. In the example shown in FIG. 10, in the pre-update flow image 65, the number of compounds is relatively large in the evaluation path leading from the "classification based on partial structure", through the "first trained model", to the "negative". On the other hand, in the flow image 64A, the number of compounds is relatively large in the evaluation path leading from the "classification based on partial structure", through the "second trained model", to the "positive".

As described above, in a case where a change has occurred in the evaluation path, it is easy to identify the path in which the change has occurred by comparing the pre-update flow image 65 with the flow image 64A. In addition, in each of the pre-update flow image 65 and the flow image 64A, the display aspect of the evaluation path is changed according to the number of compounds that have passed through the evaluation path. As a result, for the compound whose evaluation path has changed, it is easy to compare the evaluation paths before and after the change.

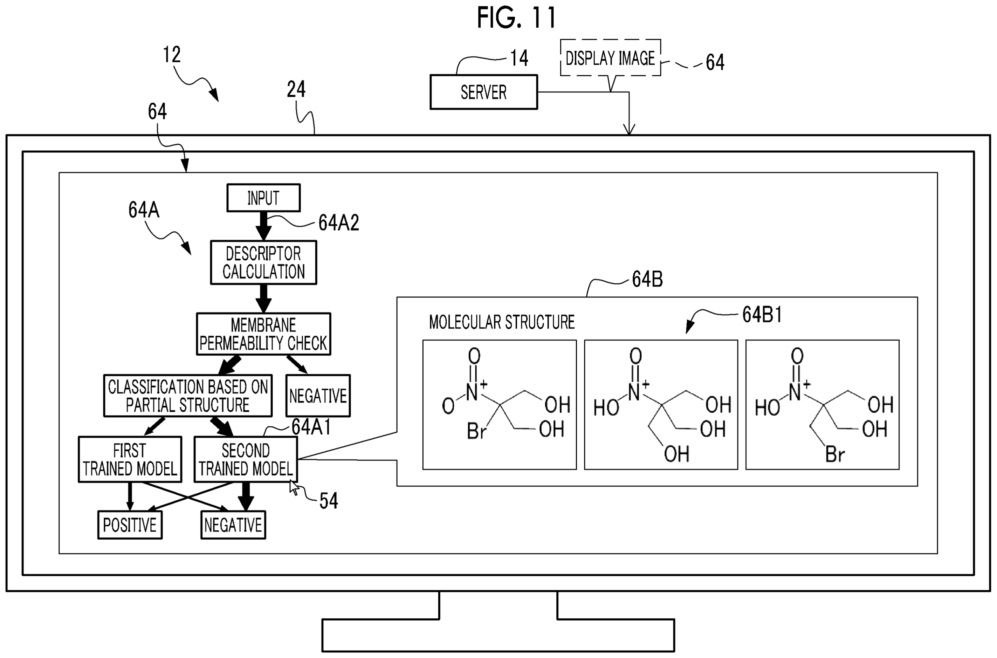

The image generation unit 30G outputs the generated display image 64 to the output unit 30H. As shown in FIG. 11 as an example, the output unit 30H of the server 14 executes control of outputting the display image 64 to the client terminal 12. In other words, the server 14 transmits information indicating the display image 64 to the client terminal 12. On the display device 24 of the client terminal 12, a screen 24A including the display image 64 is displayed.

As described above, the display image 64 includes the flow image 64A. In the toxicity evaluation using the toxicity determination flow 33, a change has occurred between the evaluation path before the change in the determination condition and the evaluation path after the change in the determination condition for some compounds (for example, the above-described compound A) in the compound group. In the flow image 64A, the path in which the change has occurred is displayed in a distinguishable manner. In the example shown in FIG. 11, the evaluation path through which the compound whose evaluation path has changed passes in the updated toxicity determination flow 33 is shown in the flow image 64A showing the updated toxicity determination flow 33.

In the example shown in FIG. 11, in the flow image 64A, the step symbol 64A1 is selected via the pointer 54. In this case, a detailed image 64B is displayed. The detailed image 64B includes a compound image 64B1 showing the compound that has passed through the selected processing step in the toxicity evaluation. Here, the step symbol 64A1 corresponding to the processing step of the toxicity evaluation using the second trained model is selected, and structural formulae showing molecular structures of three compounds that are the targets of the toxicity evaluation using the second trained model are shown in the compound image 64B1. As structural data other than the structural formula, for example, a compound graph or text describing the molecular structure may be displayed. The compound image 64B1 is an example of "structural data representing a molecular structure of a target compound" according to the technology of the present disclosure.

Here, an example of a form in which the structural data is shown in the compound image 64B1 has been described, but this is merely an example. For example, a name, a CAS number, and/or a molecular formula of the compound may be shown instead of the structural data or together with the structural data.

In addition, here, an example of a form in which, in a case where the step symbol 64A1 is selected, the detailed image 64B of the compound that has passed through the processing step corresponding to the step symbol 64A1 is displayed has been described, but this is merely an example. In a case where the step symbol 64A1 is selected, the compound input to the processing step corresponding to the step symbol 64A1 or the compound output from the processing step may be displayed in the detailed image 64B. In this case, the compound input to the processing step or the compound output from the processing step may be displayed in the detailed image 64B in a switchable manner.

In addition, here, a form of an example in which the step symbol 64A1 is selected has been described, but the connecting line 64A2 may be selected. In this case, the compound image 64B1 corresponding to the compound that has passed through the connecting line 64A2 in the toxicity evaluation is displayed.

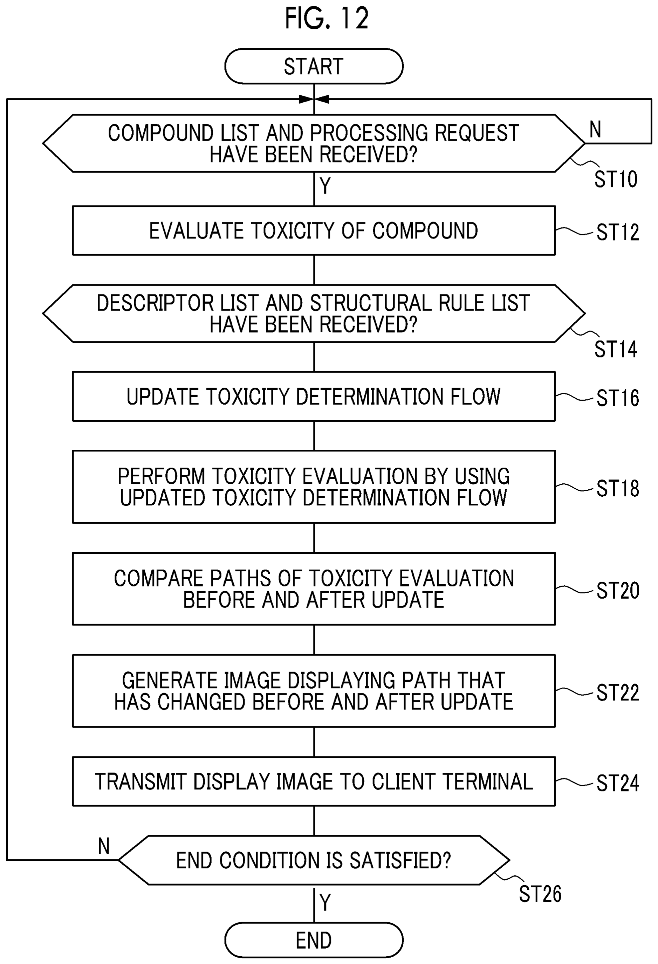

Next, control processing of the server 14 according to the present embodiment will be described with reference to FIG. 12. The server control processing is processing executed by the server 14, and includes the above-described toxicity evaluation processing, difference derivation processing, and image generation processing. FIG. 12 is a flowchart showing an example of the server control processing. The flow of the processing shown in FIG. 12 is an example of an "information processing method" according to the technology of the present disclosure.

In the server control processing shown in FIG. 12 as an example, first, in step ST10, the first acquisition unit 30A determines whether or not the compound list 58 and the processing request have been acquired. In a case where it is determined in the determination in step ST10 that the compound list 58 and the processing request have been acquired, the determination is affirmative, and the server control processing proceeds to step ST12. In a case where it is determined in the determination in step ST10 that the compound list 58 and the processing request have not been acquired, the determination is negative, and the server control processing returns to step ST10.

In step ST12, the toxicity evaluation unit 30B performs the toxicity evaluation on the compounds indicated by the compound list 58 acquired in step ST10 by using the toxicity determination flow 33. As a result, the pre-update evaluation information 62A is obtained. After the process of step ST12 is executed, the server control processing proceeds to step ST14.

In step ST14, the first acquisition unit 30A determines whether or not the descriptor list 60 and the structural rule list 61 have been acquired. In a case where it is determined in the determination in step ST14 that the descriptor list 60 and the structural rule list 61 have been acquired, the determination is affirmative, and the server control processing proceeds to step ST16. In a case where it is not determined in the determination in step ST14 that the descriptor list 60 and the structural rule list 61 have been acquired, the determination is negative, and the server control processing returns to step ST14.

In step ST16, the condition setting unit 30C updates the toxicity determination flow 33 based on the descriptor list 60 and the structural rule list 61 acquired in step ST14. Specifically, the determination condition is changed in the determination step of the toxicity determination flow 33 according to the descriptor list 60 and the structural rule list 61. After the process of step ST16 is executed, the server control processing proceeds to step ST18.

In step ST18, the toxicity evaluation unit 30B performs the toxicity evaluation on the compounds indicated by the compound list 58 acquired in step ST10 by using the updated toxicity determination flow 33. As a result, the post-update evaluation information 62B is obtained. After the process of step ST18 is executed, the server control processing proceeds to step ST20.

In step ST20, the difference derivation unit 30E compares the pre-update evaluation information 62A obtained in step ST12 with the post-update evaluation information 62B obtained in step ST18. As a result, the difference evaluation information 62C indicating the compound whose toxicity evaluation path has changed before and after the update is obtained. After the process of step ST20 is executed, the server control processing proceeds to step ST22.

In step ST22, the image generation unit 30G generates the display image 64 based on the difference evaluation information 62C. The display image 64 includes the flow image 64A. In the flow image 64A, the evaluation path after the change is shown for a compound whose evaluation path indicated by the difference evaluation information 62C has changed. After the process of step ST22 is executed, the server control processing proceeds to step ST24.

In step ST24, the output unit 30H executes control of outputting the display image 64 generated by the image generation unit 30G in step ST22 to the client terminal 12. Specifically, the output unit 30H transmits the display image 64 to the client terminal 12. After the process of step ST24 is executed, the server control processing proceeds to step ST26.

In step ST26, the output unit 30H determines whether or not a condition (hereinafter, referred to as an "end condition") for ending the server control processing is satisfied. Examples of the end condition include a condition in which an instruction to end the server control processing is received. In step ST26, in a case where the end condition is not satisfied, the determination is negative, and the server control processing proceeds to step ST10. In step ST26, in a case where the end condition is satisfied, the determination is affirmative, and the server control processing ends.

As described above, in the information processing system 10 according to the present first embodiment, the toxicity evaluation of the compound is performed by the toxicity evaluation unit 30B in the processor 30 of the server 14. In addition, the condition setting unit 30C updates the toxicity determination flow 33 based on the descriptor list 60 and the structural rule list 61. Then, the toxicity evaluation unit 30B uses the updated toxicity determination flow 33 to evaluate the toxicity of the compound. The difference derivation unit 30E generates the difference evaluation information 62C by comparing the pre-update evaluation information 62A with the post-update evaluation information 62B. The image generation unit 30G generates the flow image 64A based on the difference evaluation information 62C. In the flow image 64A, a path in which a change has occurred between the path before the change of the determination condition and the path after the change of the determination condition is displayed in a distinguishable manner. Then, the output unit 30H executes control of outputting the display image 64 including the flow image 64A. As a result, in a case where the determination condition in the toxicity evaluation is changed, it is easy to visually understand, on the flow image 64A, which path a compound that has changed in the evaluation path after the change has followed. As a result, it is easy for the user to understand the influence of the change of the determination condition on the evaluation result.

For example, in software known in the related art, the user estimates the influence on the entire flow by comparing output results between the processing steps present one after the other in time series, but it is not easy to understand the influence of the change of the determination condition on the evaluation result at first glance. In the present configuration, it is possible to visually understand, on the toxicity determination flow 33, which path a compound whose evaluation path has changed has followed. As a result, it is easy for the user to understand the influence of the change of the determination condition on the evaluation result.

In addition, in the information processing system 10 according to the present first embodiment, in the flow image 64A, the display aspect of the path in which a change has occurred is changed according to the number of compounds that have passed through the path in which a change has occurred in the toxicity evaluation. As a result, it is easy to understand, on the toxicity determination flow 33, which path a compound that has changed in the evaluation result after the change of the determination condition has followed.

In addition, in the information processing system 10 according to the present first embodiment, the flow image 64A includes a plurality of step symbols 64A1 and connecting lines 64A2 that connect the step symbols 64A1 arranged one after the other in time series. In addition, in the flow image 64A, the path is indicated by the connecting lines 64A2. The thickness of the connecting line 64A2 is changed according to the number of compounds that have passed through the path in which a change has occurred. As a result, it is easy to visually understand the number of compounds that have passed through the path in which a change has occurred.

In addition, in the information processing system 10 according to the present first embodiment, in the flow image 64A, the thickness of the connecting line 64A2 increases as the number of compounds that have passed through the path in which a change has occurred increases. The fact that the number of compounds that have passed through the path in which a change has occurred is large means that the influence of the change of the determination condition is large. As a result, by visually recognizing the path in which the connecting line is thick, it is possible to understand the path in which the influence of the change of the determination condition is large. That is, it is easy to visually understand the path in which the influence of the change of the determination condition is large.

In addition, in the information processing system 10 according to the present first embodiment, in a case where the step symbol 64A1 is selected in the flow image 64A, the compound image 64B1 showing the compound that has passed through the step symbol 64A1 is displayed in the toxicity evaluation. As a result, it is possible to understand a compound whose evaluation path has changed after the change of the determination condition for each processing step of the flow image 64A.

In addition, in the information processing system 10 according to the present first embodiment, the compound image 64B1 includes the structural data representing the molecular structure of the compound. As a result, it is possible to understand what molecular structure the compound whose evaluation path has changed after the change of the determination condition has.

The flow of the toxicity evaluation in the toxicity determination flow 33 shown in the present first embodiment is merely an example. The flow of the toxicity evaluation in the toxicity determination flow 33 can be appropriately selected or changed by the user. In addition, the processing steps shown in the flow image 64A do not necessarily have to be all of the processing steps included in the toxicity determination flow 33, and may be in a form in which some of the processing steps are displayed. For example, the processing step of interest of the user 18 may be displayed in the flow image 64A.

### (First Modification Example)

In the first embodiment, a form of an example in which the structural data representing the molecular structure of the compound that has passed through the step symbol 64A1 is included in the detailed image 64B has been described, but the technology of the present disclosure is not limited to this. In the present first modification example, structural data representing a common structure common among the compounds that have passed through the processing step is included in the detailed image 64B together with the compound image 64B1.

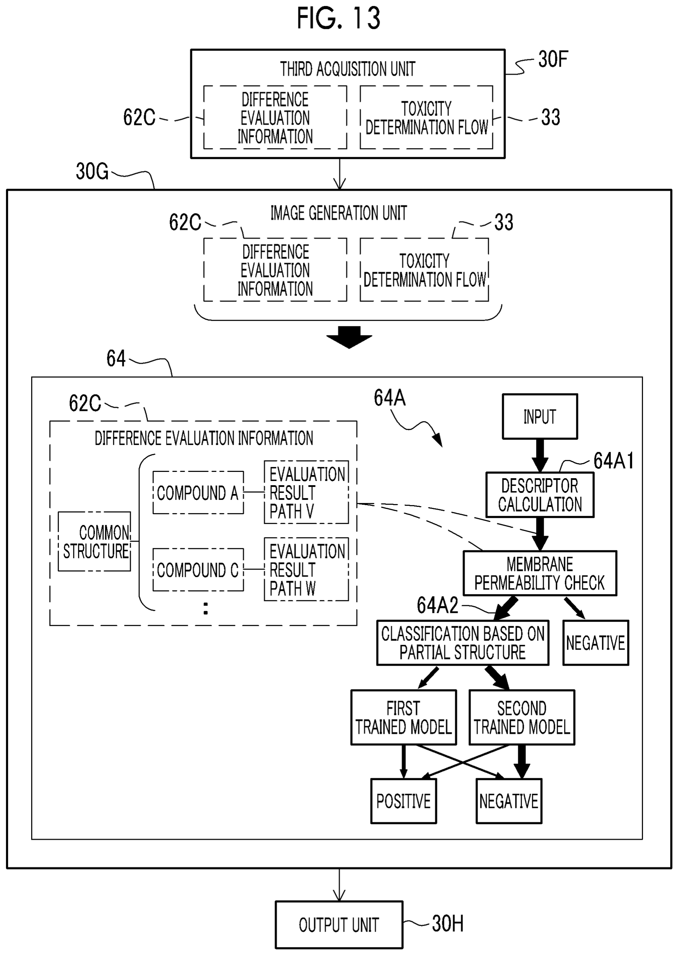

As an example, as shown in FIG. 13, the third acquisition unit 30F outputs the difference evaluation information 62C and the toxicity determination flow 33 to the image generation unit 30G. The image generation unit 30G generates a display image 64 based on the difference evaluation information 62C and the toxicity determination flow 33. The display image 64 includes a flow image 64A that is an image showing the toxicity determination flow 33.

In the flow image 64A, the step symbol 64A1 is associated with information indicating the compound that has passed through the processing step corresponding to the step symbol 64A1 based on the difference evaluation information 62C. In addition, each connecting line 64A2 is also associated with information indicating the compound that has passed through the connecting line 64A2 based on the difference evaluation information 62C. In addition, a common structure that is a structure common among the plurality of compounds (here, the compound A and the compound C) is associated with the compounds in the difference evaluation information 62C. As a result, the step symbol 64A1 and the connecting line 64A2 are associated not only with the information indicating the compound but also with the information indicating the common structure of the compound.

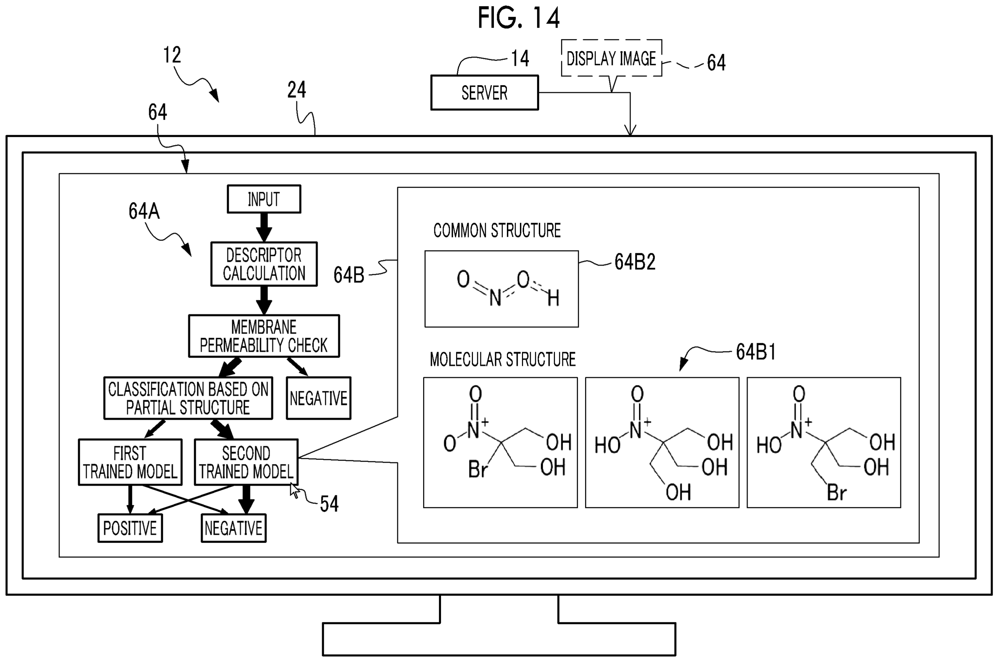

The image generation unit 30G outputs the generated display image 64 to the output unit 30H. As shown in FIG. 14 as an example, the output unit 30H of the server 14 executes control of outputting the display image 64 to the client terminal 12. In the example shown in FIG. 14, in the flow image 64A, the step symbol 64A1 is selected via the pointer 54. In this case, a detailed image 64B is displayed.

The detailed image 64B includes the compound image 64B1 and a common structure image 64B2. The common structure image 64B2 is an image showing the common structure of the plurality of compounds that have passed through the processing step. The common structure is a partial structure included as a part of the compound that has passed through the processing step, and is a structure common to the plurality of compounds.

Here, the step symbol 64A1 corresponding to the processing step of the toxicity evaluation using the second trained model is selected, and structural formulae showing the common structures of the compounds that are the targets of the toxicity evaluation using the second trained model are shown in the common structure image 64B2. The common structure image 64B2 is an example of "structural data representing a common structure" according to the technology of the present disclosure.

As described above, in the information processing system 10 according to the present first modification example, the detailed image 64B includes the common structure image 64B2 that shows the structural data representing the common structure of the compounds that have passed through the processing step. As a result, it is possible to understand what common structure a plurality of compounds whose evaluation paths have changed after the change of the determination condition have. In addition, for example, by understanding the common molecular structure, it becomes easier to consider what common structure is the cause of the effect of the change in the evaluation path due to the change in the determination condition.

Here, an example of a form in which the step symbol 64A1 is selected has been described, but the connecting line 64A2 may be selected. In this case, the common structure image 64B2 corresponding to the compound that has passed through the connecting line 64A2 in the toxicity evaluation is displayed.

### [Second Embodiment]

In the first embodiment, although an example of a form in which the toxicity evaluation processing, the difference derivation processing, and the image generation processing are performed in the server 14 has been described, the technology of the present disclosure is not limited to this. In the present second embodiment, the toxicity evaluation processing and the difference derivation processing are executed in the server 14, and the image generation processing is executed in the client terminal 12. In the present embodiment, the client terminal 12 is an example of an "information processing apparatus".

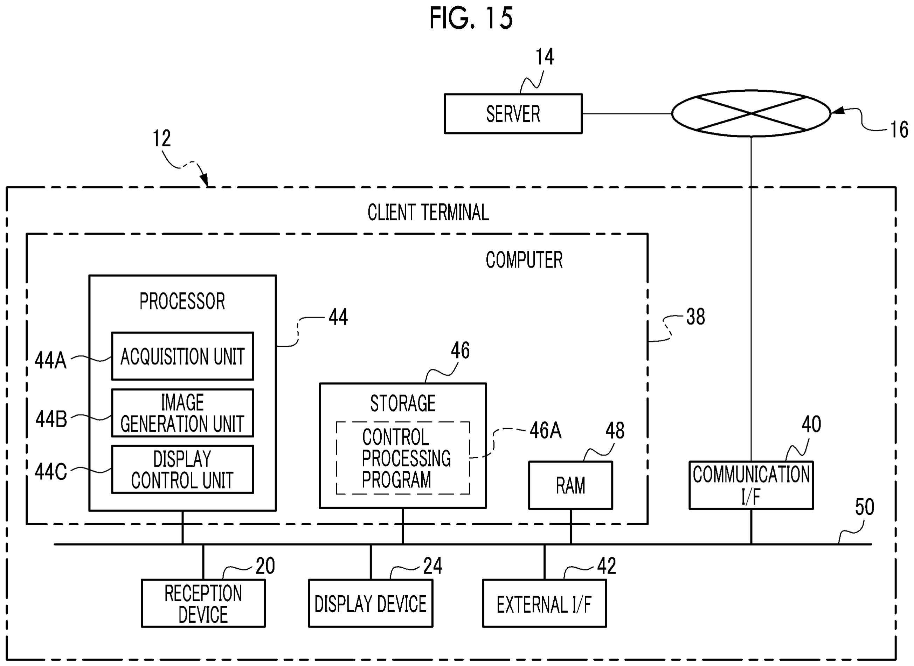

As shown in FIG. 15 as an example, the client terminal 12 comprises a computer 38, the reception device 20, the display device 24, a communication I/F 40, an external I/F 42, and a bus 50. The computer 38 comprises a processor 44, a storage 46, and a RAM 48. The processor 44, the storage 46, the RAM 48, the reception device 20, the display device 24, the communication I/F 40, and the external I/F 42 are connected to the bus 50. In the present embodiment, the processor 44 is an example of a "processor" according to the technology of the present disclosure.

In addition, since a hardware configuration (that is, the processor 44, the storage 46, and the RAM 48) of the computer 38 is basically the same as the hardware configuration of the computer 26, a description of the hardware configuration of the computer 38 will be omitted here.

The communication I/F 40 is connected to the network 16. The communication I/F 40 is responsible for transmitting and receiving information to and from an external communication device (for example, the server 14) via the network 16. For example, the communication I/F 40 transmits information in response to a request from the processor 44 to the external communication device via the network 16. In addition, the communication I/F 40 receives the information transmitted from the external communication device, and outputs the received information to the processor 44 via the bus 50.

The external I/F 42 is responsible for transmitting and receiving various types of information to and from an external device (not shown) present outside the client terminal 12. The external device may be, for example, at least one of a smart device, a personal computer, a server, a universal serial bus (USB) memory, a memory card, or a printer. An example of the external I/F 42 is a USB interface. The external device is connected directly or indirectly to the USB interface.

A control processing program 46A is stored in the storage 46. The control processing program 46A is a program for executing image generation and display control. The processor 30 executes image generation processing by reading out the control processing program 46A from the storage 46 and executing the read-out control processing program 46A on the RAM 48. The image generation processing is realized by the processor 44 operating as an acquisition unit 44A, an image generation unit 44B, and a display control unit 44C. In the present embodiment, the computer 38 is an example of a "computer" according to the technology of the present disclosure, and the control processing program 46A is an example of a "program" according to the technology of the present disclosure.

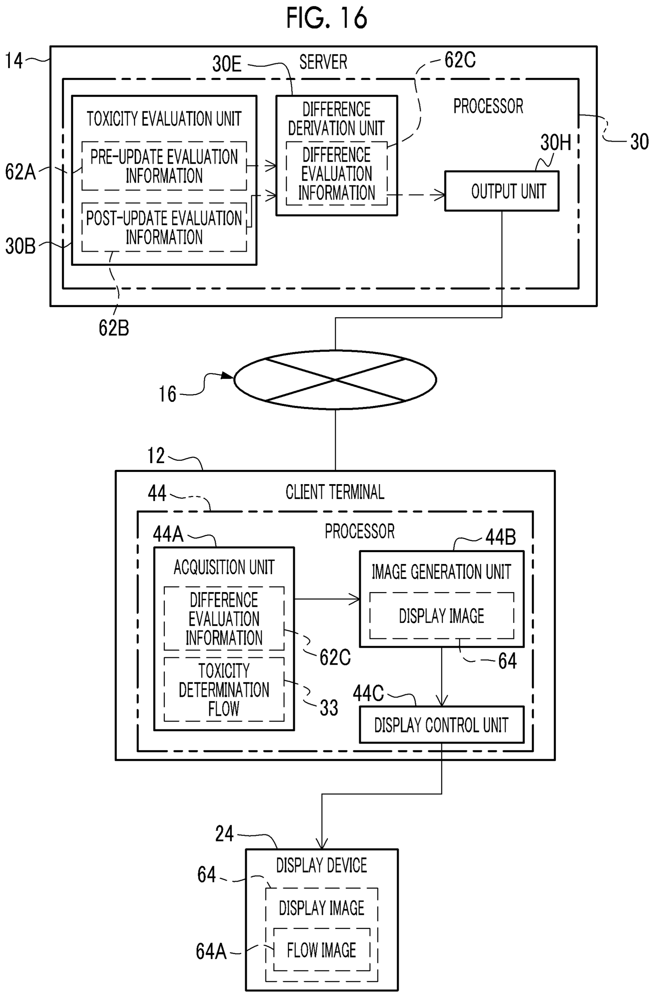

As shown in FIG. 16 as an example, in the processor 30 of the server 14, the toxicity evaluation unit 30B outputs the pre-update evaluation information 62A and the post-update evaluation information 62B obtained by the toxicity evaluation processing to the output unit 30H. In addition, the difference derivation unit 30E executes the difference derivation processing and outputs the difference evaluation information 62C obtained based on the pre-update evaluation information 62A and the post-update evaluation information 62B to the output unit 30H. The output unit 30H outputs the difference evaluation information 62C to the client terminal 12 via the network 16.

In the processor 44 of the client terminal 12, the acquisition unit 44A acquires the difference evaluation information 62C via the network 16. In addition, the acquisition unit 44A acquires the toxicity determination flow 33. Then, the acquisition unit 44A outputs the difference evaluation information 62C and the toxicity determination flow 33 to the image generation unit 44B. The image generation unit 44B generates a display image 64 based on the difference evaluation information 62C and the toxicity determination flow 33. Then, the image generation unit 44B outputs the generated display image 64 to the display control unit 44C. The display control unit 44C performs graphical user interface (GUI) control for displaying the display image 64 including the flow image 64A, thereby causing the display device 24 to display the display image 64 including the flow image 64A.

### [Third Embodiment]

In the first embodiment, although an example of a form in which the toxicity evaluation processing, the difference derivation processing, and the image generation processing are performed in the server 14 has been described, the technology of the present disclosure is not limited to this. In the present third embodiment, the toxicity evaluation processing, the difference derivation processing, and the image generation processing are performed in the client terminal 12. In the present embodiment, the client terminal 12 is an example of an "information processing apparatus".

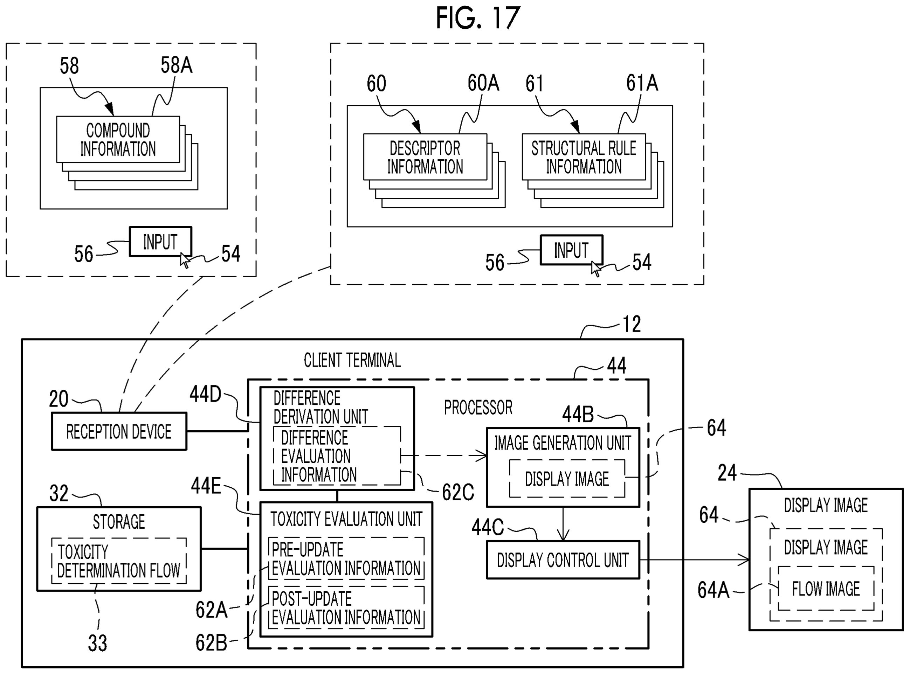

As shown in FIG. 17 as an example, the compound list 58 is received in the client terminal 12 via the reception device 20. In the processor 44 of the client terminal 12, a toxicity evaluation unit 44E performs the toxicity evaluation on the compound group indicated by the compound list 58 by using the toxicity determination flow 33. In addition, in the client terminal 12, the descriptor list 60 and the structural rule list 61 are received via the reception device 20. The toxicity determination flow 33 is updated based on the descriptor list 60 and the structural rule list 61. The toxicity evaluation unit 44E performs the toxicity evaluation on the compound indicated by the compound list 58 by using the updated toxicity determination flow 33.

In addition, a difference derivation unit 44D generates the difference evaluation information 62C based on the pre-update evaluation information 62A and the post-update evaluation information 62B. Then, the image generation unit 44B generates a display image 64 based on the difference evaluation information 62C and the toxicity determination flow 33. The display control unit 44C causes the display device 24 to display the display image 64 including the flow image 64A.

In each of the above-described embodiments, an example of a form in which the determination condition of the determination step of the toxicity determination flow 33 is changed by inputting the descriptor list 60 and the structural rule list 61 has been described, but the technology of the present disclosure is not limited to this. For example, an aspect may be adopted in which the determination condition in the toxicity determination flow 33 is changed by being directly input by the user.

In addition, in the above-described embodiments, although an example of a form in which the pre-update evaluation information 62A is obtained by executing the toxicity evaluation processing has been described, the technology of the present disclosure is not limited to this. For example, the server 14 or the client terminal 12 may receive already obtained pre-update evaluation information 62A (for example, pre-update evaluation information 62A obtained as a result of processing by an external device or pre-update evaluation information 62A obtained in the past), and perform the image generation processing.

In addition, in the above-described embodiments, although an example of a form in which the evaluation processing program 32A, the derivation processing program 32B, and the generation processing program 32C are stored in the storage 32 has been described, the technology of the present disclosure is not limited to this. For example, the evaluation processing program 32A, the derivation processing program 32B, and the generation processing program 32C may be stored in a portable storage medium such as an SSD or a USB memory. The storage medium is a non-transitory computer-readable storage medium. The evaluation processing program 32A, the derivation processing program 32B, and the generation processing program 32C stored in the storage medium are installed in the computer 26. The processor 30 executes control processing of the server 14 in accordance with the evaluation processing program 32A, the derivation processing program 32B, and the generation processing program 32C. Similarly, the control processing program 46A may be stored in a storage medium instead of the storage 46.

In the above-described embodiments, the computers 26 and 38 are exemplified, but the technology of the present disclosure is not limited to this, and a device including an application specific integrated circuit (ASIC), a field programmable gate array (FPGA), and/or a programmable logic device (PLD) may be applied instead of the computers 26 and 38. In addition, instead of the computers 26 and 38, a hardware configuration and a software configuration may be used in combination.

The following various processors can be used as hardware resources for executing the various kinds of processing described in the above-described embodiments. Examples of the processor include a CPU that is a general-purpose processor that functions as a hardware resource for executing the software, that is, the program to execute the toxicity evaluation processing, the difference derivation processing, and/or the image generation processing (hereinafter, also simply referred to as "various kinds of processing"). Examples of the processor also include a dedicated electronic circuit that is a processor whose dedicated circuit configuration is specially designed to execute specific processing, such as an FPGA, a PLD, or an ASIC. Any processor includes a memory built therein or connected thereto, and any processor uses the memory to execute various kinds of processing.

The hardware resource for executing the various kinds of processing may be configured by one of the various processors or by a combination of two or more processors of the same type or different types (for example, a combination of a plurality of FPGAs or a combination of a processor and an FPGA). Further, the hardware resource for executing the various kinds of processing may be one processor.

A first example of the configuration in which the hardware resource is configured by one processor is an aspect in which one processor is configured by a combination of one or more processors and software, and this processor functions as the hardware resource for executing the various kinds of processing. Second, as represented by system-on-a-chip (SoC), there is a form in which a processor that realizes the functions of the entire system including a plurality of hardware resources for executing the various kinds of processing with a single integrated circuit (IC) chip is used. As described above, the various kinds of processing are implemented by using one or more of the various processors as the hardware resource.

Further, specifically, an electronic circuit in which circuit elements, such as semiconductor elements, are combined can be used as the hardware structure of these various processors. The various kinds of processing are merely examples. Accordingly, it is possible to delete an unnecessary step, add a new step, or change a processing order without departing from the gist of the present disclosure.

The content of the above description and the content of the drawings are detailed explanations of the parts relating to the technology of the present disclosure, and are merely examples of the technology of the present disclosure. For example, description related to the above configurations, functions, actions, and effects is description related to an example of configurations, functions, actions, and effects of the parts relating to the technology of the present disclosure. Thus, it is needless to say that unnecessary portions may be deleted, new elements may be added, or replacement may be made to the content of the above description and the content of the drawings without departing from the gist of the technology of the present disclosure. In order to avoid complication and easily understand the parts relating to the technology of the present disclosure, in the content of the above description and the content of the drawings, the description regarding common general technical knowledge which is not necessarily particularly described in terms of embodying the technology of the present disclosure is omitted.

In the present specification, "A and/or B" is synonymous with "at least one of A or B". That is, "A and/or B" may refer to A alone, B alone, or a combination of A and B. In addition, in the present specification, in a case in which three or more matters are expressed with the connection of "and/or", the same concept as "A and/or B" is applied.

All documents, patent applications, and technical standards mentioned in the present specification are incorporated herein by reference to the same extent as in a case in which each document, each patent application, and each technical standard are specifically and individually described by being incorporated by reference.

In regard to the embodiments described above, the following appendices will be further disclosed.

### <Appendix 1>

An information processing apparatus comprising:
a processor,
in which the processor executes control of outputting a flowchart that indicates a procedure for obtaining a toxicity evaluation result for a plurality of target compounds, which are targets of a toxicity evaluation, and that includes a plurality of processing steps set in advance and arranged in time series, the processing steps including a determination processing step in which a determination condition is changeable and a subsequent path branches according to a determination result, and
in a case where, after the toxicity evaluation result is obtained according to the procedure for at least one of the plurality of target compounds, the determination condition in the determination processing step is changed and the toxicity evaluation result is obtained according to the procedure under the changed determination condition,
in the control of outputting the flowchart, in a case where a change occurs between an unchanged path taken to reach the toxicity evaluation result before the change in the determination condition and a changed path taken to reach the toxicity evaluation result after the change in the determination condition, the processor displays the path in which the change has occurred in a distinguishable manner.

### <Appendix 2>

The information processing apparatus according to Appendix 1,
in which, in a case where the toxicity evaluation result is obtained for the plurality of target compounds before and after the change in the determination condition,
in the flowchart, a display aspect of the path in which the change has occurred is changed according to the number of the target compounds that have passed through the path in which the change has occurred in the toxicity evaluation.

### <Appendix 3>

The information processing apparatus according to Appendix 2,
in which the flowchart shows the plurality of processing steps and connecting lines that connect the processing steps arranged one after the other in time series, and shows the path by using the connecting lines, and
the display aspect is a thickness of the connecting line.

### <Appendix 4>

The information processing apparatus according to Appendix 3,
in which the thickness of the connecting line increases as the number of the target compounds that have passed through the path in which the change has occurred increases.

### <Appendix 5>

The information processing apparatus according to any one of Appendices 1 to 4,
in which the flowchart shows the plurality of processing steps and connecting lines that connect the processing steps arranged one after the other in time series, and shows the path by using the connecting lines, and
in a case where the processing step or the connecting line shown in the flowchart is selected, an image showing the target compound that has passed through the path shown by the processing step or the connecting line in the toxicity evaluation is displayed.

### <Appendix 6>

The information processing apparatus according to Appendix 5,
in which the image includes structural data representing a molecular structure of the target compound.

### <Appendix 7>

The information processing apparatus according to Appendix 6,
in which the image includes structural data representing a common structure that is a partial structure included as a part of the target compound and that is a structure common among the plurality of target compounds that have passed through the selected processing step or connecting line.

The disclosure of JP2023-091157 filed on June 1, 2023 is incorporated herein by reference in its entirety. All documents, patent applications, and technical standards mentioned in the present specification are incorporated herein by reference to the same extent as in a case in which each document, each patent application, and each technical standard are specifically and individually described by being incorporated by reference.

## Claims

1. An information processing apparatus comprising a processor, wherein:
the processor is configured to execute control of outputting a flowchart that indicates a procedure for obtaining a toxicity evaluation result for a plurality of target compounds, which are targets of a toxicity evaluation, and that includes a plurality of processing steps set in advance and arranged in time series, the processing steps including a determination processing step in which a determination condition is changeable and a subsequent path branches according to a determination result, and
in a case where, after the toxicity evaluation result is obtained according to the procedure for at least one of the plurality of target compounds, the determination condition in the determination processing step is changed and the toxicity evaluation result is obtained according to the procedure under the changed determination condition,
in the control of outputting the flowchart, in a case where a change occurs between an unchanged path taken to reach the toxicity evaluation result before the change in the determination condition and a changed path taken to reach the toxicity evaluation result after the change in the determination condition, the processor is configured to display the path in which the change has occurred in a distinguishable manner.

2. The information processing apparatus according to claim 1, wherein, in a case where the toxicity evaluation result is obtained for the plurality of target compounds before and after the change in the determination condition,
in the flowchart, a display aspect of the path in which the change has occurred is changed according to the number of the target compounds that have passed through the path in which the change has occurred in the toxicity evaluation.

3. The information processing apparatus according to claim 2, wherein:
the flowchart shows the plurality of processing steps and connecting lines that connect the processing steps arranged one after the other in time series, and shows the path by using the connecting lines, and
the display aspect is a thickness of the connecting line.

4. The information processing apparatus according to claim 3, wherein the thickness of the connecting line increases as the number of the target compounds that have passed through the path in which the change has occurred increases.

5. The information processing apparatus according to claim 1, wherein:
the flowchart shows the plurality of processing steps and connecting lines that connect the processing steps arranged one after the other in time series, and shows the path by using the connecting lines, and
in a case where the processing step or the connecting line shown in the flowchart is selected, an image showing the target compound that has passed through the path shown by the processing step or the connecting line in the toxicity evaluation is displayed.

6. The information processing apparatus according to claim 5, wherein the image includes structural data representing a molecular structure of the target compound.

7. The information processing apparatus according to claim 6, wherein the image includes structural data representing a common structure that is a partial structure included as a part of the target compound and that is a structure common among the plurality of target compounds that have passed through the selected processing step or connecting line.

8. An information processing method comprising:
executing control of outputting a flowchart that indicates a procedure for obtaining a toxicity evaluation result for a plurality of target compounds, which are targets of a toxicity evaluation, and that includes a plurality of processing steps set in advance and arranged in time series, the processing steps including a determination processing step in which a determination condition is changeable and a subsequent path branches according to a determination result,
wherein, in a case where, after the toxicity evaluation result is obtained according to the procedure for at least one of the plurality of target compounds, the determination condition in the determination processing step is changed and the toxicity evaluation result is obtained according to the procedure under the changed determination condition,
in the control of outputting the flowchart, in a case where a change occurs between an unchanged path taken to reach the toxicity evaluation result before the change in the determination condition and a changed path taken to reach the toxicity evaluation result after the change in the determination condition, the path in which the change has occurred is displayed in a distinguishable manner.

9. A program for causing a computer to execute a process comprising:
executing control of outputting a flowchart that indicates a procedure for obtaining a toxicity evaluation result for a plurality of target compounds, which are targets of a toxicity evaluation, and that includes a plurality of processing steps set in advance and arranged in time series, the processing steps including a determination processing step in which a determination condition is changeable and a subsequent path branches according to a determination result,
wherein, in a case where, after the toxicity evaluation result is obtained according to the procedure for at least one of the plurality of target compounds, the determination condition in the determination processing step is changed and the toxicity evaluation result is obtained according to the procedure under the changed determination condition,
in the control of outputting the flowchart, in a case where a change occurs between an unchanged path taken to reach the toxicity evaluation result before the change in the determination condition and a changed path taken to reach the toxicity evaluation result after the change in the determination condition, the path in which the change has occurred is displayed in a distinguishable manner.
